# EUROPÄISCHE PATENTSCHRIFT

(11) **EP 0 757 983 B1**
(45) Veröffentlichungstag und Bekanntmachung des Hinweises auf die Patenterteilung: **13.10.1999**
(21) Anmeldenummer: 96111978.1
(22) Anmeldetag: 25.07.1996
(51) Int. Cl.: C07C 291/04

(54) **Verfahren zur Herstellung von tertiären Aminoxiden**
Process for the preparation of tertiary amine oxides
Procédé de préparation d'aminoxides tertiaires

(30) Priorität: 07.08.1995 DE 19528945
(43) Veröffentlichungstag der Anmeldung: 12.02.1997
(73) Patentinhaber: Clariant GmbH, 65929 Frankfurt am Main (DE)
(72) Erfinder: Bäder, Georg, Dr., 65719 Hofheim (DE); Tamargo, Ramon Joglar, 43001 Tarragona (ES)

(56) Entgegenhaltungen:
- EP-A- 0 037 996

## Beschreibung

Die Umsetzung von tertiären Aminen, z.B. vom Typ Fettalkyldimethylamin oder Di-Fettalkyl-methylamin, mit einer wäßrigen Wasserstoffperoxidlösung ergibt die entsprechenden tertiären Aminoxide. Derartige Aminoxide sind nicht wasserhärteempfindliche, gutschäumende, wenig hautreizende Tenside und werden deshalb als Bestandteile von Reinigungsmitteln und Körperpflegemitteln verwendet.

Die bisher bekannten Verfahren zur Herstellung von tertiären Aminoxiden betreffen die Verbesserung der Ausbeute, die Verringerung des Gehaltes an Nitrosamin sowie das Vermeiden von störenden Gelphasen bei der Herstellung.

Bei dem Verfahren aus US-A-4 247 480 werden hohe Ausbeuten an tertiärem Aminoxid erzielt, indem die Oxidation des tertiären Amins mit einer wäßrigen Wasserstoffperoxidlösung in Gegenwart von 0,01 bis 2 Gew.-% Kohlendioxid, bezogen auf das eingesetzte Amin und gegebenenfalls in Gegenwart von Tetraacetylethylendiamin, dessen Salz, Polyphosphaten, Stannaten, einem Hydroxycarbonsäuresalz oder dem Salz einer Polycarbonsäure erfolgt. Bevorzugt wird eine 5 bis 70 gew.- %ige wäßrige Wasserstoffperoxidlösung eingesetzt, wobei entweder eine zu dem Amin stöchiometrische Menge oder bevorzugt ein Überschuß von 5 bis 10 % an Wasserstoffperoxid verwendet wird. Die Umsetzung erfolgt bei einer Temperatur im Bereich von 40 bis 80C.

Zur Verbesserung der Ausbeute wird in US-A-3 283 007 der Einsatz einer geringen Menge Pentaacetyldiethylentriamin während der Oxidation tertiärer, mit Schwermetallen verunreinigter Amine empfohlen. Die eingesetzte Wasserstoffperoxidlösung besitzt eine Konzentration von mindestens 20 Gew.-% Wasserstoffperoxid, bevorzugt 30 bis 75 Gew.-% und die Reaktionstemperatur liegt im Bereich von 40 bis 80C.

Mit dem Verfahren nach US-A-4 889 954 lassen sich tertiäre Amine in hohen Ausbeuten zu den entsprechenden Aminoxiden mit einem geringen Gehalt an Nitrosaminen umsetzen, wobei die Oxidation des tertiären Amins in Gegenwart eines Dialkylcarbonsäureesters als Katalysator und gegebenenfalls Ascorbinsäure als Cokatalysator erfolgt. Geeignete wäßrige Wasserstoffperoxidlösungen besitzen eine Konzentration von 3 bis 90 Gew.-% Wasserstoffperoxid. Erforderlich ist eine mindestens stöchiometrische Menge an Wasserstoffperoxid, bevorzugt werden 1 bis 5 Mol, insbesondere 1 bis 1,5 Mol Wasserstoffperoxid pro Mol tertiärem Amin. Die Umsetzungstemperatur ist in einem weiteren Temperaturbereich wählbar, üblicherweise in einem Bereich von 0 bis 100C.

Zur Vermeidung der während der Herstellung von tertiären Aminoxiden auftretenden Gelphasen werden verschiedene Lösungsmöglichkeiten im Stand der Technik beschrieben.

Nach US-A-3 215 741 wird das Auftreten von Gelphasen bei der Verwendung von Wasserstoffperoxidlösungen mit einer Konzentration im Bereich von 20 bis 90 Gew.-% Wasserstoffperoxid und bei einer Reaktionstemperatur im Bereich von 40 bis 80C beobachtet und können durch gleichzeitige Zugabe einer ausreichenden Menge Wasser während der Oxidation des tertiären Amins vermieden werden.

Gemäß US-A-3 432 555 kann die Bildung von Gelphasen vermieden werden, indem die Mischung aus Amin, Wasser und einem Komplexbildner, z.B. Pentaacetyldiethylentriamin, zunächst auf eine Temperatur von 85 bis 115C erhitzt wird und anschließend mindestens die stöchiometrische Menge einer wäßrigen Wasserstoffperoxidlösung bei exothermen Temperaturanstieg zugesetzt wird.

Zur Vermeidung der Gelbildung bei der Herstellung von Di-C₆-C₂₀-Alkylmethylaminoxid erfolgt entsprechend EP-A-0 230 510 die Oxidation des zugrundeliegenden tertiären Amins mit mindestens der stöchiometrischen Menge einer mindestens 40 gew.-%igen Wasserstoffperoxidlösung.

DE-A-30 14 510 beschreibt ein Verfahren zur Herstellung von Naphthenoylamino-alkylendialkyl-aminoxiden, bei dem man das zugrundeliegende Amin in Form einer wäßrigen, feinteiligen 20 bis 40 gew.-%igen Dispersion, die gegebenenfalls 0,5 bis 5 Gew.-%, bezogen auf die Dispersion, des entsprechenden Aminoxids enthält, bei einer Temperatur oberhalb 70C mit einer sauerstoffabspaltenden Verbindung, z.B. wäßriger Wasserstoffperoxidlösung oxidiert. Es hat sich gezeigt, daß die Verwendung einer wäßrigen, feinteiligen Dispersion der Amine es gestattet, die Reaktionszeit, die für die Oxidation benötigt wird, stark abzukürzen. Zur zusätzlichen Stabilisierung der Dispersion können 0,5 bis 5 Gew.-% einer wäßrigen Lösung des herzustellenden Aminoxids hinzugefügt werden.

Bei der Herstellung von tertiären Aminoxiden kann bei Anspringen der Reaktion eine starke Schaumbildung auftreten. Damit ergibt sich sowohl die Gefahr des Überschäumens infolge der exothermen Reaktion und auch die Gefahr der unkontrollierten Zersetzung des eingesetzten Wasserstoffperoxids.

Aufgabe der vorliegenden Erfindung ist es, ein Verfahren zur Herstellung von tertiären Aminoxiden zur Verfügung zu stellen, ohne die vorstehend genannten Nachteile der unzureichenden Ausbeute, der Gel- und der Schaumbildung.

Überraschenderweise hat sich gezeigt, daß durch Zusatz von tertiärem Aminoxid bei der Oxidation eines tertiären Amins mit Wasserstoffperoxid, Alkalihypochlorit, anorganischen oder organischen Persäuren das Auftreten der vorgenannten Nachteile während des Herstellverfahrens unterbunden werden kann.

Gegenstand der vorliegenden Erfindung ist ein Verfahren zur Herstellung von tertiären Aminoxiden der allgemeinen Formel I 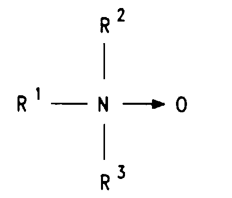 worin
- R¹: C₁-C₃-Alkyl,
- R²: C₁-C₂₀-Alkyl und
- R³: C₆-C₂₂-Alkyl bedeuten,
durch Umsetzung eines tertiären Amins der allgemeinen Formel II

R¹R²R³N

mit Wasserstoffperoxid, Alkalihypochlorit oder anorganischen oder organischen Persäuren, das dadurch gekennzeichnet ist, daß die Umsetzung in Gegenwart von 1 bis 15 Gew.-%, bezogen auf den Reaktionsansatz, eines tertiären Aminoxids der Formel I, bevorzugt des herzustellenden tertiären Aminoxids erfolgt.

Bei den Aminen der Formel II bedeuten R¹ bevorzugt Methyl oder Ethyl, R² C₁-C₁₂-Alkyl und R³ C₈-C₂₀-Alkyl.
Beispielhaft seien genannt: Octyldimethylamin, Dodecyldimethylamin, Tetradecyldimethylamin, Hexadecyldimethylamin, Octadecyldimethylamin, Eicosyldimethylamin, Dioctylmethylamin, Didecylmethylamin und Didodecylmethylamin.
Die Amine der Formel II können als Einzelverbindungen oder in Form einer synthetischen oder natürlichen Mischung, wie z.B. Cocosdimethylamin, eingesetzt werden.

Für die Oxidation werden Wasserstoffperoxid, Alkalihypochlorit, anorganische oder organische Persäuren verwendet. Bevorzugt ist die Verwendung einer wäßrigen Wasserstoffperoxidlösung. Die Konzentration der wäßrigen Wasserstoffperoxidlösung ist in einem breiten Bereich wählbar und umfaßt Lösungen mit einer Konzentration im Bereich von 3 bis 90 Gew.-% Wasserstoffperoxid. Bevorzugt beträgt die Konzentration 20 bis 70 Gew.-%, insbesondere 20 bis 40 Gew.-% Wasserstoffperoxid.
Die eingesetzte Menge an Wasserstoffperoxid entspricht zumindest der stöchiometrischen Menge an eingesetztem Amin. Bevorzugt werden 1 - 1,5 Mol Wasserstoffperoxid pro Mol Amin, besonders bevorzugt 1,01 - 1,1 Mol der Formel II. Etwaiger Überschuß an Wasserstoffperoxid kann nach erfolgter Umsetzung durch Zugabe eines Reduktionsmittels oder eines Wasserstoffperoxid zerstörenden Mittels beseitigt werden. Abhängig von der Reinheit des eingesetzten Amins können erforderlichenfalls Komplexbildner wie EDTA, zugesetzt werden.

Die erfindungsgemäße Umsetzung kann in einem weiten Temperaturbereich durchgeführt werden. Die Temperatur ist so zu wählen, daß einerseits eine zufriedenstellende Umsetzungsrate erzielt wird und andererseits die eingesetzten Ausgangsstoffe bzw. die erhaltenen Produkte nicht zersetzt werden. Üblicherweise erstreckt sich der Temperaturbereich von 0 - 100C, bevorzugt 30 - 90C, besonders bevorzugt von 45 - 85C.

Das erfindungsgemäße Verfahren kann in den üblichen, in der chemischen Industrie verwendeten Rührbehältern durchgeführt werden. Im Hinblick auf die Tatsache, daß die bei den bisher üblichen Verfahren aufgetretene Schaumbildung unterbleibt, ergeben sich vielfältige Ausgestaltungen für das erfindungsgemäße Verfahren. So ist es möglich, Wasserstoffperoxid, Alkalihypochlorit oder anorganische oder organische Persäuren bei erhöhter Temperatur im Bereich von 60 - 90C gefahrlos zu dem Amin und dem Aminoxid zuzugeben. Deren Zugabe kann sowohl auf einmal als auch in Teilmengen erfolgen.

In einer anderen Verfahrensvariante werden Amin, Aminoxid und Wasserstoffperoxid, Alkalihypochlorit oder anorganische oder organische Persäuren vorgelegt und auf die erforderliche Reaktionstemperatur hochgeheizt. Der Reaktionsstart erfolgt hierbei kontrolliert, Schaum- und Gelbildung treten nicht auf.

Im Verlauf der Oxidation entsteht eine klare bis schwach opaliszierende Lösung des Aminoxids der Formel I. Je nach Gehalt der Lösungen an Aminoxid weisen die Lösungen unterschiedliche Viskosität auf, die als dünnflüssig bis mittelviskos einzustufen ist. Gelartige bis hochviskose Lösungen werden nicht gebildet. Vorteilhafterweise tritt bei der Durchführung des erfindungsgemäßen Verfahrens keine Schaumbildung auf, was wiederum eine kontrollierte Reaktionsführung sowie die optimale Nutzung der verwendeten Reaktionskessel im Hinblick auf eine möglichst große Menge an Ausgangsstoffen gestattet. Des weiteren wird mit dem erfindungsgemäßen Verfahren die Bildung von tertiären Aminoxiden mit vermindertem Nitrosamingehalt erzielt.

### Herstellungsbeispiele

### Beispiel 1

3948 kg Dimethylmyristilamin, 9078 kg Wasser, 1000 kg Dimethyllaurylaminoxid (25 gew.-%ig) und 2 kg EDTA werden im Rührkessel gemischt und auf 80C geheizt und anschließend 1612 kg wäßrige Wasserstoffperoxidlösung (35 gew.-%ig) innerhalb von 2 Stunden zugegeben. Die Reaktionsmischung wird über einen Zeitraum von 8 Stunden bei einer Temperatur von 80 bis 82C gehalten.
Während der Umsetzung treten weder Schaumbildung noch Gelbildung auf. Aus diesem Grund liegt die umgesetzte Stoffmenge zu 50 % über der im Vergleich durchgeführten Umsetzung ohne Aminoxidzugabe. Die Reaktionszeit liegt um 30 % unterhalb der beim entsprechenden Vergleichsversuch benötigten Zeitspanne. Der Nitrosamingehalt ist um 50 % niedriger. Der Gehalt an nicht umgesetztem Amin bzw. Wasserstoffperoxid ist mit 0,5 % bzw. 0,05 % sehr gering. Der für die Umsetzung erforderliche Wasserstoffperoxidüberschuß beträgt 1 %.

### Beispiel 2

Es werden 2570 kg Dimethyllaurylamin, 5000 kg Wasser, 2 kg EDTA, 500 kg Dimethyllaurylaminoxid und 1100 kg wäßrige Wasserstoffperoxidlösung (35 gew.-%ig) bei ca. 30C gemischt und mit einer Temperaturrate von 0,5C/min. aufgeheizt. Die Reaktion startet kontrolliert bei ca. 45 C mit einem kontrollierbaren Temperaturanstieg von 0,3C/min. Die Umsetzung erfolgt unter Kühlung bei der vorgenannten Temperatur. Schaumbildung und Gelbildung treten nicht auf.
In einem entsprechend durchgeführten Vergleichsversuch ohne Zugabe von Aminoxid springt die Reaktion erst bei 10C höherer Temperatur mit einer mehr als doppelten Geschwindigkeit an, so daß eine kontrollierte Reaktionsführung nicht gewährleistet ist.

### Beispiel 3

Durchführung entsprechend Beispiel 2.
Die Zugabe der wäßrigen Wasserstoffperoxidlösung erfolgt hälftig bei einer Temperatur von 60C. Die Reaktion startet problemlos unter kontrollierbarem Temperaturanstieg auf ca. 85C in ca. 1,5 Stunden. Nach Abkühlen auf ungefähr 60C wird die zweite Hälfte an Wasserstoffperoxidlösung zugegeben. Schaum- sowie Gelbildung sind nicht zu beobachten. Der eingesetzte Kessel kann in vollem Umfang genutzt werden.

## Patentansprüche

1. Verfahren zur Herstellung von tertiären Aminoxiden der allgemeinen Formel I 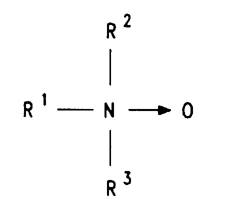 worin
R¹ C₁-C₃-Alkyl,
R² C₁-C₂₀-Alkyl und
R³ C₆-C₂₂-Alkyl bedeuten,
durch Umsetzung eines tertiären Amins der allgemeinen Formel II
R¹R²R³N
mit Wasserstoffperoxid, Alkalihypochlorid oder anorganischen oder organischen Persäuren, dadurch gekennzeichnet, daß die Umsetzung in Gegenwart von 1 bis 15 Gew.-%, bezogen auf den Reaktionsansatz, eines tertiären Aminoxids der Formel I erfolgt.

2. Verfahren nach Anspruch 1, dadurch gekennzeichnet, daß die Herstellung in Gegenwart des herzustellenden tertiären Aminoxids erfolgt.

3. Verfahren nach Anspruch 1 oder 2, dadurch gekennzeichnet, daß R¹ Methyl oder Ethyl, R² C₁-C₁₂-Alkyl und R³ C₈-C₂₀-Alkyl bedeuten.

4. Verfahren nach einem der Ansprüche 1 bis 3, dadurch gekennzeichnet, daß eine wäßrige Wasserstoffperoxidlösung eingesetzt wird.

5. Verfahren nach Anspruch 5, dadurch gekennzeichnet, daß eine 3 bis 90 gew.-%ige, bevorzugt 20 bis 70 gew.-%ige, besonders bevorzugt 20 bis 40 gew.-%ige Wasserstoffperoxidlösung eingesetzt wird.

6. Verfahren nach einem der Ansprüche 1 bis 6, dadurch gekennzeichnet, daß 1 - 1,5 Mol, bevorzugt 1,01 - 1,1 Mol Wasserstoffperoxid pro Mol Amin der Formel II eingesetzt werden.

7. Verfahren nach einem der Ansprüche 1 bis 7, dadurch gekennzeichnet, daß die Umsetzung bei einer Temperatur im Bereich von 0 bis 100°C, bevorzugt von 30 - 90°C, besonders bevorzugt von 45 - 85°C erfolgt.

## Claims

1. A process for the preparation of a tertiary amine oxide of the formula I 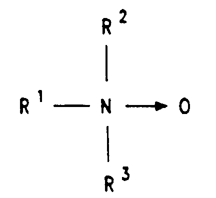 in which
R¹ is C₁-C₃-alkyl,
R² is C₁-C₂₀-alkyl and
R³ is C₆-C₂₂-alkyl,
by reaction of a tertiary amine of the formula II
R¹R²R³N
with hydrogen peroxide, alkali metal hypochlorite or inorganic or organic peracids, which comprises carrying out the reaction in the presence of 1 to 15% by weight, based on the reaction mixture, of a tertiary amine oxide of the formula I.

2. The process as claimed in claim 1, wherein the preparation is carried out in the presence of the tertiary amine oxide to be prepared.

3. The process as claimed in claim 1 or 2, wherein R¹ is methyl or ethyl, R² is C₁-C₁₂-alkyl and R³ is C₈-C₂₀-alkyl.

4. The process as claimed in one of claims 1 to 3, wherein an aqueous hydrogen peroxide solution is employed.

5. The process as claimed in claim 4, wherein a 3 to 90% strength by weight, preferably 20 to 70% strength by weight, particularly preferably 20 to 40% strength by weight, hydrogen peroxide solution is employed.

6. The process as claimed in one of claims 1 to 5, wherein 1- 1.5 mol, preferably 1.01 - 1.1 mol, of hydrogen peroxide are employed per mole of amine of the formula II.

7. The process as claimed in one of claims 1 to 6, wherein the reaction is carried out at a temperature in the range from 0 to 100 C, preferably from 30 to 90 C, particularly preferably from 45 to 85 C.

## Revendications

1. Procédé de préparation d'oxydes d'amines tertiaires de formule générale I 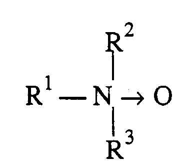 dans laquelle
R¹ représente un groupe alkyle en C₁-C₃,
R² représente un groupe alkyle en C₁-C₂₀, et
R³ représente un groupe alkyle en C₆-C₂₂,
par réaction d'une amine tertiaire de formule générale II
R¹R²R³N
avec du peroxyde d'hydrogène, un hypochlorite alcalin ou des peracides inorganiques ou organiques, caractérisé en ce qu'on effectue la réaction en présence de 1 à 15% en poids, par rapport à la charge réactionnelle, d'un oxyde d'amine tertiaire de formule I.

2. Procédé selon la revendication 1, caractérisé en ce que l'on effectue la préparation en présence de l'oxyde d'amine tertiaire à préparer.

3. Procédé selon la revendication 1 ou 2, caractérisé en ce que R¹ représente un groupe méthyle ou éthyle, R² représente un groupe alkyle en C₁-C₁₂ et R³ un groupe alkyle en C₈-C₂₀.

4. Procédé selon l'une des revendications 1 à 3, caractérisé en ce qu'on utilise une solution aqueuse de peroxyde d'hydrogène.

5. Procédé selon la revendication 4, caractérisé en ce qu'on utilise une solution de peroxyde d'hydrogène à 3 à 90% en poids, de préférence à 20 à 70% en poids, de manière particulièrement préférée à 20 à 40% en poids.

6. Procédé selon l'une des revendications 1 à 5, caractérisé en ce qu'on utilise 1 - 1,5 mol, de préférence 1,01 - 1,1 mol de peroxyde d'hydrogène par mol d'amine de formule II.

7. Procédé selon l'une des revendications 1 à 6, caractérisé en ce qu'on effectue la réaction à une température dans le domaine de 0 à 100°C, de préférence de 30 - 90°C, de manière particulièrement préférée de 45 - 85°C.
